# EUROPEAN PATENT APPLICATION

(11) **EP 2 526 974 A1**
(43) Date of publication of application: **28.11.2012**
(21) Application number: 11732816.1
(22) Date of filing: 14.01.2011
(51) Int. Cl.: A61L 2/16, A01N 25/02, A01N 25/34, A01N 59/12, C11D 1/722, C11D 3/10, C11D 3/20, C11D 3/48, C11D 7/04, C11D 7/12, C11D 7/26, C11D 17/00, C11D 17/06, G02C 13/00

(54) **NUCLEATED TABLET FOR CONTACT LENS CLEANING, CONTACT LENS CLEANING PREPARATION CONTAINING SAME, AND METHOD FOR CLEANING CONTACT LENS**

(30) Priority: 18.01.2010 JP 2010008387
(71) Applicant: Ophtecs Corporation, Hyogo 650-0047 (JP)
(72) Inventor: SASANUMA, Kazuhiro, Hyogo 650-0047 (JP); TONE, Kazuhiro, Hyogo 650-0047 (JP)
(74) Representative: Matthews, Derek Peter
(86) International application number: PCT/JP2011/000182
(87) International publication number: WO 2011/086944

(57) **Abstract**

An object of the present invention is to provide a nucleated tablet for contact lens (CL) cleaning that makes it possible to disinfect a CL simply and easily in the presence of a foaming component; a CL cleaning preparation that contains the nucleated tablet; and a method for cleaning a CL. The nucleated tablet for CL cleaning of the invention is a nucleated tablet including an outer shell that contains iodine-based bactericide, a carbonate, an organic acid, and a surfactant having, per partial weight of 2000 out of the molecular weight thereof, less than one hydroxyl group, and an inner nucleus that contains a neutralizing agent, a carbonate, an organic acid, and a surfactant, wherein the inner nucleus is coated for extended release.

## Description

### [Technical Field]

The present invention relates to a nucleated tablet for contact lens cleaning that makes it possible to attain sufficient sterilization through a iodine-based bactericide in the presence of sodium carbonate, which is a foaming component, and that is allowed only to stand still in a lens case which is a closed container, thereby taking care of a contact lens; and a contact lens cleaning preparation that contains such a nucleated tablet for cleaning. The invention relates to a method for cleaning a contact lens by the use of the nucleated tablet for contact lens cleaning.

### [Background Art]

Examples of an effective eyesight-correcting method at present include the use of eyeglasses, the wearing of contact lenses (hereinafter abbreviated to CLs), and surgical operations (such as LASIK operation). Of these methods, CLs are used by many people since the CLs have a high eyesight-correcting power, the use thereof gives a wider visual field through corrected-eyesight than the use of eyeglasses, a stable visual field is obtained even at the time of exercises, and further the use thereof is lower in invasiveness than any surgical operation. At present, in Japan, about 16, 000, 000 people, which correspond to about 13% of the population of Japan, use CLs.

When a CL is worn or unworn or is continuously used, microorganisms such as bacteria, and proteins or lipids originating from fingers or lacrimal fluid are caused to adhere onto the CL. In a case where such microorganisms or stains are left, the CL is deformed, or foreign object feeling or an itch is generated when the CL is worn so that a deterioration in contact-lens wearing feeling is caused. In addition, the microorganisms may cause an eye infection, or the cornea or conjunctive may cause a trouble against the surface of the eye ball. For this reason, when the same CL is used plural times, it is indispensable to take a care, such as the disinfection thereof by heat or with a chemical agent, or the cleaning thereof with a surfactant, a protease and/or a lipid degrading enzyme. In the case of, in particular, a water-containing contact lens, which is in general called a soft contact lens and used, microorganisms propagate easily since the material thereof contains water. Thus, when the CL is taken a care, it is essential to disinfect microorganisms thereon.

A main method for the disinfection of a CL is classified into two types, i.e., boiling disinfection, and chemical disinfection. In recent years, the chemical disinfection has been mainly used since an instrument exclusive thereto is not required, and operations therefor are simple and easy.

Disinfectants for the chemical disinfection are of some types. Main examples thereof include preparations each containing hydrogen peroxide as a disinfecting component; preparations each containing, as a disinfecting component, a sterilizing compound having in the molecule thereof an electrically charged functional group, a typical example thereof being biguanide; and preparations each containing iodine as a disinfecting component. Of these examples, a preparation for CL cleaning that contains iodine as a disinfecting component has a very strong disinfecting power, and the preparation gives only a small stimulus to an eye and creates no danger even when the preparation is incorrectly used. Thus, attention has been paid to this preparation.

As a disinfectant containing iodine as a disinfectant component, use is widely made of povidone iodine, which is a complex that is generally called iodophor, is composed of a polymer and a iodine molecule and is polyvinyl pyrrolidone to which iodine is coordinated. As a one-liquid type combined preparation for disinfecting, neutralizing and cleaning a contact lens, for example, Patent Literature 1 discloses a preparation made of a combination of a first preparation which contains a iodine-based disinfectant and a protease with a second preparation which contains a sulfur-containing reducing agent and a foaming agent and has a coat for extended release, in which at least one of the first and second preparations contains a nonionic surfactant.

As a method for stabilizing a iodine-based bactericide, known are a method of incorporating a iodide salt into a solution of povidone iodine (Patent Literature 2), a method of incorporating an alkalifying agent thereinto, and storing the incorporated material in a glass bottle (Patent Literature 3), and a method of storing a iodine solution in a container made of polyester resin (Patent Literature 4).

### [Citation List]

### [Patent Literatures]

PTL 1: Japanese Laid-Open Patent Application Publication No. 09-111298
PTL 2: US Patent No. 4,996,048
PTL 3: Japanese Laid-Open Patent Application Publication No. 05-70356
PTL 4: Japanese Laid-Open Patent Application Publication No. 2001-117056

### [Summary of Invention]

### [Technical Problem]

The combined preparation disclosed in Patent Literature 1 is composed of the first preparation and the second preparation; when a CL is disinfected therewith, it is necessary to dissolve these two preparations in an aqueous solution for dissolution. However, the preparation containing an iodine-based bactericide, which is the first preparation, contains no foaming agent; thus, in order to dissolve the preparation in a homogeneous form, a stirring operation is required. Thus, the combined preparation is complicated in operation for use.

An object of the present invention is to provide a nucleated tablet for CL cleaning that makes it possible to disinfect a CL simply and easily in the presence of a foaming component; a CL cleaning preparation that contains the nucleated tablet; and a method for cleaning a CL.

### [Solution to Problem]

The present inventors have made eager researches to solve the problems. As a result, the inventors have found out that in the case of preparing a nucleated tablet which includes an outer shell containing a iodine-based bactericide and a foaming agent, and an inner nucleus containing a neutralizing agent, a foaming agent, and a surfactant of a specific type, a CL can be safely disinfected without making any stirring operation only by dissolving a solid preparation, which is a single preparation, into a dissolving liquid. Thus, the invention has been accomplished.

Specifically, the present invention relates to a nucleated tablet for contact lens cleaning, including:
an outer shell that contains an iodine-based bactericide, a carbonate, an organic acid, and a surfactant having, per partial weight of 2000 out of the molecular weight thereof, less than one hydroxyl group; and
an inner nucleus that contains a neutralizing agent, a carbonate, an organic acid, and a surfactant, wherein
the inner nucleus is coated for extended release.

By incorporating the carbonate and the organic acid into the outer shell, foam of carbon dioxide is generated when the nucleated tablet is immersed in a dissolving liquid. As a result, without making any stirring operation, the iodine-based bactericide is homogeneously dissolved in the dissolving liquid so that a CL can be effectively disinfected.

According to the present invention, after the period when the CL is disinfected with the iodine-based bactericide is sufficiently continued, the inner nucleus can be dissolved in the dissolving liquid. The inner nucleus also contains, as a foaming agent, the carbonate and the organic acid, thereby making it possible to dissolve the neutralizing agent homogeneously in the dissolving liquid, without making any stirring operation, by the generation of foam of carbon dioxide.

As a result, when a CL is set together with the nucleated tablet and the dissolving liquid in a lens case, the disinfection of the CL with the iodine-based bactericide and the neutralization of the iodine-based bactericide after the disinfection can be finished only by allowing the lens case to stand still as it is. Thus, operation for the disinfection can be made simple and easy.

The carbonate may be sodium carbonate, or potassium carbonate. The organic acid may be an oxocarboxylic acid such as citric acid or tartaric acid. The content of the carbonate and the organic acid in the outer shell is preferably from 5 by weight or more to 60% by weight.

The inner nucleus is coated for extended release, thereby making it possible to adjust the period until the inner nucleus starts to be dissolved after the dissolution of the outer shell, so that a sufficient period for disinfection with the iodine-based bactericide can be kept. The coating for extended release can be attained by coating the surface of the inner nucleus with hydroxypropylcellulose (HPC), hydroxypropylmethylcellulose (HPMC) or hydroxyethylcellulose (HEC).

The surfactant can exhibit an advantageous effect of promoting the collapse of the outer shell. The surfactant is high in solubility in any dissolving liquid to prevent secondary pollution of a CL surface, which is caused by the adhesion of residues thereto. The surfactant may be a nonionic surfactant such as polysolvate or polyethylene glycol, or an anionic surfactant. The content of the surfactant in the outer shell is preferably from 0.2 by weight or more to 30 by weight or less.

The iodine-based bactericide may be povidone iodine, polyvinyl alcohol iodine or cyclodextrin iodine. The iodine-based bactericide is preferably povidone iodine since the chemical agent is versatile and inexpensive.

The surfactant used in the present invention is a surfactant having, per partial weight of 2000 out of the number-average molecular weight thereof, less than one hydroxyl group (more than zero).

The present inventors and the like have made investigations to render a preparation containing an iodine-based bactericide a foaming preparation in order to improve the convenience. However, the incorporation of a foaming component and a surfactant into the preparation containing an iodine-based bactericide has caused the iodine-based bactericide, which is a disinfecting component, to be unstabilized and deteriorated in disinfecting effect in some cases. Patent Literatures 2 to 4 never disclose any method for stabilizing the iodine-based bactericide in such a foaming preparation. Thus, the inventors have made further investigations about a method in which while the stability of a preparation is maintained, a foaming component is incorporated thereinto.

As a result, the present inventors have made it clear that a cause for making the iodine-based bactericide unstable is water generated by esterification reaction between the surfactant and the organic acid, which is used together with the carbonate as the foaming agent. The inventors have then found out that this esterification reaction between the organic acid and the surfactant is easily caused when the surfactant has, per partial weight of 2000 out of the number-average molecular weight thereof, one or more hydroxyl groups.

The surfactant contained in the outer shell and having, per partial weight of 2000 out of the molecular weight thereof, less than one hydroxyl group is preferably polyethylene glycol 6000. The content of the iodine-based bactericide in the outer shell is preferably from 0.2 by weight or more to 30 by weight or less.

The incorporation of povidone iodine as the iodine-based bactericide into the outer shell has caused troubles, such as sticking when a tablet is made, or poor dissolution of the outer shell in a dissolving liquid when a tablet is immersed in the dissolving liquid, in some cases. The present inventors and the like have investigated methods for preventing these troubles to find out that the use of polyethylene glycol 6000 as the surfactant having, per partial weight of 2000 out of the number-average molecular weight thereof, less than one hydroxyl group, makes it possible to prevent the troubles at the time of making a tablet or dissolving a tablet.

The neutralizing agent (reducing agent) for the iodine-based bactericide may be a sulfur-containing reducing agent such as sodium sulfite or sodium thiosulfate, ascorbic acid, or edetic acid. The neutralizing agent is in particular preferably sodium sulfite.

The inner nucleus further includes a protease. The protease may be a protease produced by Bacillus microorganisms, such as subtilisin, or an enzyme originating from the pancreas of an animal, such as pancreatin.

This is for removing a protein adhering to a CL surface to improve the cleaning effect. When the protease is incorporated into the inner nucleus, it is preferred to incorporate a pH adjustor into the inner nucleus or a dissolving liquid in such a manner that the pH of the dissolving liquid can be an optimum pH for the protease when the inner nucleus is dissolved. The content of the protease in the inner nucleus is preferably from 1 by weight or more to 20 by weight or less.

The present invention relates to a CL cleaning preparation, including:
the nucleated tablet for CL cleaning; and
a dissolving liquid.

Further, the present invention relates to a method for cleaning a CL, including: dissolving the nucleated tablet for CL cleaning in a dissolving liquid in a closed container; and allowing the CL, which is a cleaning target, to stand still in the state that the CL is immersed in the dissolving liquid until the nucleated tablet is completely dissolved.

### [Advantageous Effects of Invention]

According to the nucleated tablet for CL cleaning, the CL cleaning preparation, and the method for cleaning a CL of the present invention, in a case where a CL is set in a disinfecting container before sleeping, the CL can be worn at the time of rising up, and the CL can be simply and easily and certainly disinfected in daily life. Since this is attained only by dissolving the nucleated tablet, which is a single tablet, in a dissolving liquid, an accident that the constituting preparation is lost can also be prevented.

### [Brief Description of Drawings]

[Fig. 1] Figs. 1 (a) and 1 (b) are each an external view of a nucleated tablet according to the present invention.
[Fig. 2] Figs. 2(a) to 2(c) are views illustrating a method of using a CL cleaning preparation according to the present invention.
[Fig. 3] Fig. 3 is a graph for comparing effective iodine remaining quantities based on different surfactants.

### [Description of Embodiments]

Hereinafter, embodiments of the present invention are described with appropriate reference to the drawings. The invention is not limited to descriptions given below.

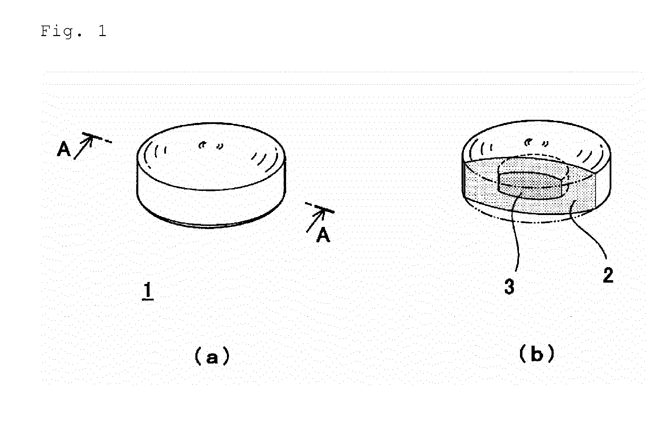

Figs. 1 (a) and 1 (b) are each an external view of a nucleated tablet 1 according to the present invention. The right view thereof is a sectional view taken on line A-A of the left view thereof. The nucleated tablet 1 is composed of an outer shell 2 and an inner nucleus 3. The shape and size of the nucleated tablet 1 are not particularly limited; however, it is sufficient that the nucleated tablet 1 contains chemical agents necessary for cleaning CLs (one set of two CLs) one time.

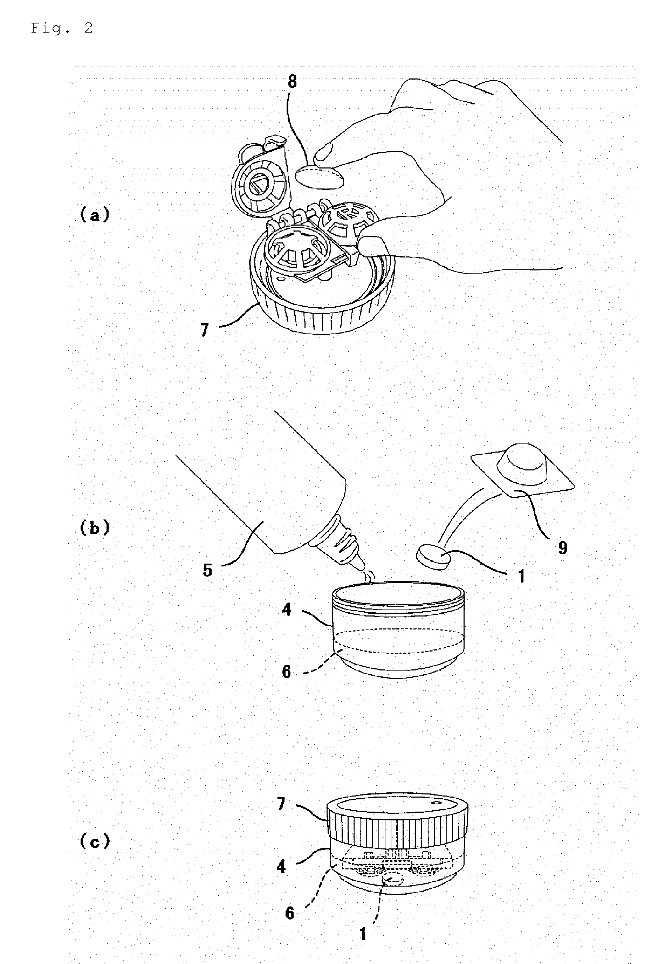

Figs. 2(a) to 2(c) are views illustrating a method of using a CL cleaning preparation according to the present invention. First, CLs 8 are set to a lens case upper part 7 (Fig. 2 (a)).

Next, a nucleated tablet 1 is taken out from a nucleated tablet package 9, and then put into a lens case lower part 4. From a dissolving liquid container 5, a predetermined volume of a dissolving liquid 6 is collected into the lens case (Fig. 2 (b)). It is preferred to mark a scale in the lens case lower part 4 in order to indicate the volume of the dissolving liquid 6 to be collected.

Next, the lens case upper part 7 is fitted to the lens case lower part 4 (Fig. 2 (c)). At this time, it is preferred to adjust the volume of the dissolving liquid 6 to cause the whole of the CLs 8 to be immersed in the dissolving liquid 6.

In this state, the lens case is allowed to stand still for 4 hours or more. The outer shell 2 of the nucleated tablet 1 is gradually dissolved in the dissolving liquid by foaming effect based on a carbonate and an organic acid (foaming caused by carbon dioxide), so that the dissolving liquid 6 is gradually colored to a color from yellow to brown by effect of an iodine-based bactericide. The iodine-based bactericide and a surfactant can be evenly dissolved in the dissolving liquid 6 by the foaming effect even when a stirring operation is not made at all.

With respect to the outer shell of the nucleated tablet 1, it is preferred to adjust the pressure for making a tablet, the amount of a disintegrating agent, or some other factor to dissolve the outer shell completely in a period less than 5 minutes and disinfect the CLs with the iodine-based bactericide. By coating the surface of the inner nucleus (coating the surface for extended release) with hydroxypropylcellulose (HPC), hydroxypropylmethylcellulose (HPMC) or hydroxyethylcellulose (HEC), the inner nucleus can be also kept not to be immediately dissolved even when the outer shell is dissolved. This manner makes it possible to maintain sufficiently a period for disinfecting the CLs with the iodine-based bactericide. It is preferred to adjust the period until the inner nucleus starts to be dissolved in accordance with the concentration of the iodine-based bactericide.

When the outer shell has been dissolved, the inner nucleus starts to be dissolved. The inner nucleus also contains a carbonate and an organic acid incorporated thereinto; thus, the incorporated components can be evenly dissolved in the dissolving liquid even when no stirring operation is made. Since the inner nucleus contains a neutralizing agent for the iodine-based bactericide incorporated thereinto, the iodine-based bactericide is gradually neutralized to the accompaniment of the dissolution of the inner nucleus. Following the neutralization, the dissolving liquid 6 is gradually made colorless.

A basic function of the inner nucleus is the release of the neutralizing agent, thereby attaining the neutralization (reduction) of the iodine-based bactericide. However, further incorporation of a surfactant makes it possible to remove stains based on a protein or fat on the surface of each of the CLs. Additional incorporation of a protease into the inner nucleus also makes it possible to remove the protein on the surface of the CL effectively. In order to adjust, at this time, the pH of the dissolving liquid to an optimal pH for the protease, it is preferred to adjust the pH of the dissolving liquid 5 or incorporate a pH adjustor into the inner nucleus.

With respect to the inner nucleus of the nucleated tablet 1, it is preferred to adjust the pressure for making a tablet and the amount of a disintegrating agent to dissolve the inner nucleus completely in a period less than 30 minutes, and finish the neutralization of the iodine-based bactericide, and the cleaning of the surface of each of the CLs. After the cleaning operation, the CLs can be taken out from the lens case upper part 7, and be worn as they are. However, when the inner nucleus contains a surfactant and/or a protease incorporated thereinto, it is preferred that after the cleaning operation the dissolving liquid 6 is thrown away, and subsequently the CLs are rinsed with a new dissolving liquid inside the lens case.

It is recommended that a CL is disinfected every day. According to the CL cleaning preparation and CL cleaning method of the present invention, only by setting a dissolving liquid, a nucleated tablet, and CLs inside a lens case before sleeping, and then leaving these members as they are, the disinfection and cleaning of the CLs are completed at the time of rising up and thus, the CL can be immediately worn.

For the CL cleaning preparation and CL cleaning method of the present invention, a dissolving liquid is necessary for dissolving the nucleated tablet for CL cleaning. The dissolving liquid preferably has an osmotic pressure of about 100 to 400 mOsm, and more preferably has an osmotic pressure of about 200 to 300 mOsm. The component(s) thereof is/are not particularly limited. The dissolving liquid is not limited about the type thereof as far as the liquid is a liquid usable as a CL cleaning liquid, for example, a physiological saline solution, a phosphoric acid solution or a boric acid solution.

Into the nucleated tablet for CL cleaning of the present invention and/or the dissolving liquid may be optionally incorporated a buffer agent, an tonicity agent, or a thickening agent as far as the sterilizing effect of the iodine-based bactericide is not hindered and any significant effect is not produced onto living body (eyeball) and the physical properties or shape of a CL.

The buffer agent is used to stabilize the pH of the dissolving liquid. The buffer agent may be a buffer agent used generally for a CL cleaning solution or for an ophthalmic preparation. It is preferred to use, for example, (1) a phosphoric acid-based buffer agent, such as sodium monohydrogenphosphate, sodium dihydrogenphosphate, potassium monohydrogenphosphate, or potassium dihydrogenphosphate; (2) a boric acid-based buffer agent, such as boric acid, or sodium borate; or (3) a tris buffer agent, such as trisaminomethane and diluted hydrochloric acid, or trismalate and a diluted sodium hydroxide solution. It is preferred to incorporate the buffer agent into the nucleated tablet for CL cleaning and/or the dissolving liquid in such a manner that when the nucleated tablet for CL cleaning is dissolved in the dissolving liquid, the concentration thereof ranges from 0.05 to 1.0% by weight.

When the nucleated tablet for CL cleaning is dissolved in the dissolving liquid, it is preferred to set the pH of the resultant solution into range of 5 to 8. If the pH is in an acidic range having a pH less than 5 or in an alkaline range having a pH more than 8, eye irritancy or ophthalmopathy may be caused. Thus, the pH should be avoided.

The tonicity agent is used to adjust the osmotic pressure of the dissolving liquid. The tonicity agent may be a tonicity agent used generally for a CL cleaning solution or an ophthalmic preparation. It is preferred to use, for example, (1) an alkali or alkaline earth metal salt, such as sodium chloride, potassium chloride or magnesium chloride; or (2) a saccharide, such as glucose, mannitol, sorbitol, xylitol, or dextran.

These tonicity agents may be used alone or in combination of two or more thereof. When the nucleated tablet for CL cleaning is dissolved in the dissolving liquid, it is preferred to adjust the concentration of the tonicity agent (s) into the range of 0.5 to 5.0% by weight. By the adjustment of the concentration of the tonicity agent(s), the osmotic pressure generated when the nucleated tablet for CL cleaning is dissolved in the dissolving liquid is adjusted into the range preferably from about 100 to 400 mOsm, and more preferably from about 200 to 300 mOsm.

The thickening agent is used to adjust the viscosity of the dissolving liquid. The thickening agent may be a thickening agent used generally for a CL cleaning solution or an ophthalmic preparation. It is preferred to use, for example, (1) a polyol, such as glycerin, ethylene glycol, propylene glycol, polyethylene glycol or polyvinyl alcohol; or (2) a saccharide, such as trehalose, sucrose, carboxymethylcellulose, hydroxyethylcellulose, or hydroxylpropylmethylcellulose. The concentration of the thickening agent is not particularly limited. When the nucleated tablet for CL cleaning is dissolved in the dissolving liquid, it is preferred to set the agent concentration into the range of 0.05 to 5.0% by weight.

### <Measurement of effective iodine remaining percentages>

Into a 50-mL vial was put a mixture of 3 g of a povidone iodine powder, 72 mg of each surfactant shown in Table 1, and 360 mg of tartaric acid. This vial was closed, and stored in a thermostat at a temperature of 40°C. With respect to each of the samples, the effective iodine remaining percentage in the povidone iodine powder was measured after 0, 3, 7 and 15 days.

**TABLE 1**

| Surfactant | Number-average molecular weight | Number of hydroxyl groups per molecule | Number of hydroxyl groups per partial weight of 2000 out of molecular weight |
|---|---|---|---|
| Polyoxyethylene (196) polyoxypropylene (67) glycol | 10000 - 15000 | 2 | 0.27 - 0.4 |
| Polyethylene glycol 4000 | 2600 - 3800 | 2 | 1.05 - 1.54 |
| Polyethylene glycol 6000 | 7300 - 9300 | 2 | 0.43 - 0.55 |
| Polyoxyl 40 stearate | 2044 | 1 | > 1 |
| Sodium lauroylsarcosine | 293.38 | 0 | 0 |

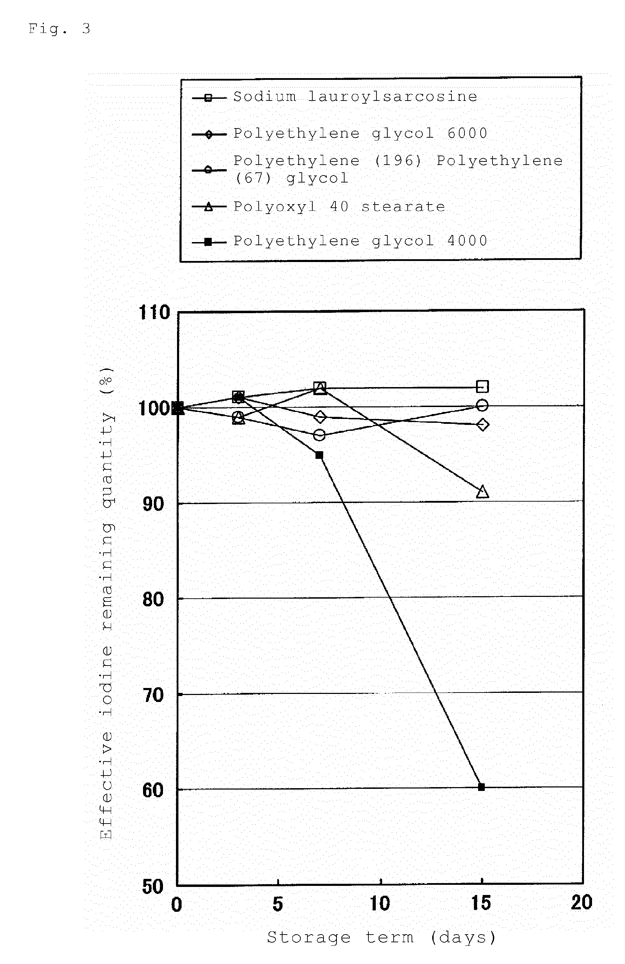

The effective iodine remaining percentage (%) was calculated by determining quantitatively the effective iodine quantities before and after storages in accordance with the effective iodine quantitative determination method described in Item "Povidone iodine" in Japanese Pharmacopoeia. Fig. 3 is a graph showing measurement results of the effective iodine remaining percentage. For polyethylene glycol 4000, the effective iodine remaining percentage was largely lowered as compared with the other surfactants. For each of sodium lauroylsarcosine, polyethylene glycol 6000, and polyoxyethylene (196) polyoxypropylene (67) glycol, the effective iodine remaining percentage after 15 days was close to 100%. For polyoxyl 40 stearate, the percentage was about 90%.

### <Production of nucleated tablets>

### [Examples 1 to 4]

Each nucleated tablet was produced which contained components shown in Table 2. A method for producing the tablet was specifically as described below. In Table 2, respective incorporated quantities denote incorporated quantities per tablet.

**TABLE 2**

| | Component name | Incorporation quantities | | | |
|---|---|---|---|---|---|
| | | Example 1 | Example 2 | Example 3 | Example 4 |
| Outer shell | Povidone iodine | 4.0 mg | 4.0 mg | 10.0 mg | 20.0 mg |
| | Dry sodium carbonate | 28.0 mg | 16.5 mg | 35.0 mg | 28.0 mg |
| | Tartaric acid | 40.0 mg | 30.0 mg | 50.0 mg | 40.0 mg |
| | D-Mannitol | Appropriate quantity | Appropriate quantity | Appropriate quantity | Appropriate quantity |
| | Macrogol 6000 | Appropriate quantity | Appropriate quantity | Appropriate quantity | Appropriate quantity |
| Inner nucleus | Dry sodium sulfite | 2.4 mg | 3.0 mg | 4.0 mg | 8.0 mg |
| | Dry sodium carbonate | 15.4 mg | 19.3 mg | 19.3 mg | 15.4 mg |
| | Tartaric acid | 10.0 mg | 18.2 mg | 12.5 mg | 10.0 mg |
| | D-Mannitol | Appropriate quantity | Appropriate quantity | Appropriate quantity | Appropriate quantity |
| | Sodium chloride | 24.0 mg | 32.0 mg | 16.0 mg | 20.0 mg |
| | Bioprase conc | 8.0 mg | 8.0 mg | 8.0 mg | 8.0 mg |
| | Polyoxyethylene polyoxypropylene glycol | 1.8 mg | 1.8 mg | 1.8 mg | 1.8 mg |
| | Hypromellose | 10.0 mg | 10.0 mg | 10.0 mg | - |

First, out of its inner nucleus components, the following were collected: dry sodium carbonate, sodium chloride (partial volume), D-mannitol (partial volume), Bioprase conc (generic name: subtilisin, manufactured by Nagase ChemteX Corporation), and polyoxyethylene polyoxypropylene glycol (partial volume). The collected components were made into the form of granules. The granules, which were white, were used as first granules.

Next, out of the inner nucleus components, the following were collected: tartaric acid, sodium chloride (the balance), D-mannitol (the balance), and polyoxyethylene polyoxypropylene glycol (the balance). The collected components were made into the form of granules. The granules, which were white, were used as second granules.

A predetermined amount of the first granules was mixed with a predetermined amount of the second granules, and the mixture was made into naked tablets (66 mg per tablet) in accordance with the item of Pharmaceutical Preparation General Rules in Japanese Pharmacopoeia. The naked tablets were coated (coated for extended release) with hypromellose in a usual manner to prepare inner nuclei.

Separately, povidone iodine, dry sodium carbonate, tartaric acid, and D-mannitol, which were outer shell components, were collected. These components were made into a granular form while Macrogol 6000 (drug listed in Japanese Pharmacopoeia) was added to the components. The granules, which were slightly yellowish-brown, were used as third granules.

A machine for making a nucleated tablet was used to make the inner nuclei and the third granules into nucleated tablets as nucleated tablets of each of Examples 1 to 4. For the nucleated tablets, their outer shells were completely dissolved in 3 minutes when 8 mL of a boric acid buffer solution was used as a dissolving liquid. For the nucleated tablets, after the dissolution of the outer shells, the inner nuclei were completely dissolved. As a result, povidone iodine was neutralized and the dissolving liquid turned transparent and colorless.

### [Example 5]

A production of nucleated tablets was attempted by conducting operations entirely identical with those in Example 1 except that polyoxyethylene (196) polyoxypropylene (67) glycol was used as a lubricant for their outer shell in the same amount. When the nucleated tablets were made, a sticking phenomenon was frequency caused. Thus, obstacles were slightly caused to the mass production of the nucleated tablets.

### [Comparable Example 1]

A production of nucleated tablets was attempted by conducting operations entirely identical with those in Example 1 except that polyoxyl 40 stearate was used as a lubricant for their outer shell in the same amount. When the nucleated tablets were made, a sticking phenomenon was frequency caused. Thus, obstacles were slightly caused to the mass production of the nucleated tablets.

### [Comparable Example 2]

A production of nucleated tablets was attempted by conducting operations entirely identical with those in Example 1 except that sodium lauroylsarcosine was used as a lubricant for their outer shell in the same amount. When the nucleated tablets were made, a sticking phenomenon was frequency caused. Thus, obstacles were slightly caused to the mass production of the nucleated tablets.

### [Industrial Applicability]

The nucleated tablet for CL cleaning of the invention and the CL cleaning preparation thereof, which contains this nucleated tablet, and the CL cleaning method thereof are useful as a CL cleaning preparation and a CL cleaning method.

### [Reference Signs List]

1: Nucleated tablet
2: Outer shell
3: Inner nucleus
4: Lens case lower part
5: Dissolving liquid bottle
6: Dissolving liquid
7: Lens case upper part
8: Contact lenses
9: Nucleated tablet

## Claims

1. A nucleated tablet for contact lens cleaning, comprising:
an outer shell that contains an iodine-based bactericide, a carbonate, an organic acid, and a surfactant having, per partial weight of 2000 out of the molecular weight thereof, less than one hydroxyl group; and
an inner nucleus that contains a neutralizing agent, a carbonate, an organic acid, and a surfactant, wherein
the inner nucleus is coated for extended release.

2. The nucleated tablet for contact lens cleaning according to claim 1, wherein the outer shell comprises povidone iodine as the iodine-based bactericide, and comprises polyethylene glycol 6000 as the surfactant.

3. The nucleated tablet for contact lens cleaning according to claim 1, wherein the neutralizing agent is sodium sulfite.

4. The nucleated tablet for contact lens cleaning according to claim 1, wherein the inner nucleus further comprises a protease.

5. A contact lens cleaning preparation, comprising:
the nucleated tablet for contact lens cleaning as recited in claim 1; and
a dissolving liquid.

6. A method for cleaning a contact lens, comprising:
dissolving the nucleated tablet for contact lens cleaning as recited in claim 1 in a dissolving liquid in a closed container; and
allowing the contact lens, which is a cleaning target, to stand still in the state that the contact lens is immersed in the dissolving liquid until the nucleated tablet is completely dissolved.
